# EUROPEAN PATENT SPECIFICATION

(11) **EP 1 606 620 B1**
(45) Date of publication and mention of the grant of the patent: **09.01.2008**
(21) Application number: 04723119.6
(22) Date of filing: 24.03.2004
(51) Int. Cl.: G01N 33/543

(54) **IMMOBILIZATION METHOD AND KIT THEREFOR**
IMMOBILISIERUNGSVERFAHREN UND KIT DAFÜR
PROCEDE ET TROUSSE D'IMMOBILISATION

(30) Priority: 25.03.2003 SE 0300805; 25.03.2003 US 457508 P
(43) Date of publication of application: 21.12.2005
(73) Proprietor: Biacore AB, 754 50 Uppsala (SE)
(72) Inventor: KARLSSON, Robert, S-754 40 Uppsala (SE); SJÖDIN, Anders, S-741 96 Knivsta (SE)
(74) Representative: Widén, Björn
(86) International application number: PCT/SE2004/000447
(87) International publication number: WO 2004/086046

(56) References cited:
- WO-A-02/072873
- WO-A1-98/04740
- WO-A1-99/29303
- WO-A2-02/082078
- SUKHORUKOV G. B., ET AL.: 'Multilayer films containing immobilized nucleic acids. Their structure and possibilities in biosensor applications' BIOSENSORS & BIOELECTRONICS vol. 11, no. 9, 1996, pages 913 - 922, XP002238541
- KOJI SODE, ET AL.: 'Application of bacterial magnetic particles for highly selective mRNA recovery system' BIOTECHNOLOGY TECHNIQUES vol. 7, no. 9, September 1993, pages 688 - 694, XP002979295

## Description

### FIELD OF THE INVENTION

The present invention relates to the preparation of solid support surfaces, and more particularly to a method of immobilizing target molecules to solid support surfaces.

### BACKGROUND OF THE INVENTION

A variety of analytical techniques are used to characterize interactions between molecules, particularly in the context of assays directed to the detection and interaction of biomolecules. For example, antibody-antigen interactions are of fundamental importance in many fields, including biology, immunology and pharmacology. In this context, many analytical techniques involve binding of a "ligand", such as an antibody, to a solid support, followed by contacting the ligand with an "analyte", such as an antigen. Following contact of the ligand and analyte, some characteristic is measured which is indicative of the interaction, such as the ability of the ligand to bind the analyte. It is often desired that after measurement of the interaction, it should be possible to dissociate the ligand-analyte pair in order to "regenerate" free ligand, thereby enabling reuse of the ligand surface for a further analytical measurement.

The binding, or immobilization, of the ligand to the support can be either direct or indirect. In direct immobilization, the ligand is coupled directly to the surface, typically covalently, whereas in indirect immobilization the ligand is captured (usually by non-covalent binding) by a molecule that is directly coupled to the surface, typically covalently. While indirect immobilization is restricted to ligands which have a suitable binding site or tag for the surface-coupled molecule, it has several advantages. For example, non-covalent immobilization of the ligand is simpler to effect than covalent coupling, and the ligand need not be pure but can be captured from a crude sample. Further, all ligands are immobilized in a known and consistent orientation on the surface. Usually, it is also possible to regenerate the ligand surface by general regeneration conditions so that repeated capture can be performed on the surface. Exemplary capture molecules include protein A and protein G which both bind to the Fc-part of immunoglobulins (antibodies), NTA metal chelates which bind to histidine tags, and oligonucleotides which hybridize to a complementary oligonucleotide tag.

The use of oligonucleotides as capture molecules, which is disclosed in more detail in e.g. US-A-5,648,213, has the additional advantage that by varying the oligonucleotide sequences very specific oligonucleotide pairs may easily be created. The specificity of capture oligonucleotides bound to discrete surface areas, or spots, on a solid support can then be used to address different ligands to the different spots. Another advantage resides in that relatively harsh conditions may be used to regenerate the oligonucleotide capture surface as compared to, for example, a protein surface which is susceptible to denaturation. A problem in immobilizing oligonucleotides to a solid support, however, is their relatively large negative charge which makes it difficult to immobilize them to negatively charged surfaces. Traditional approaches to overcome this problem, including the use of high salt concentrations to shield off the charges, or low pH conditions, have either failed or given low immobilization degrees, as will be demonstrated in comparative examples below.

In the genetic engineering field, it is previously known to use polycations as nonviral agents for delivering DNA to cells. The polycations efficiently bind to negatively charged DNA, which results in a substantial DNA compaction. Also, if the polyelectrolyte complex formed has an overall positive charge, it increases the interaction with a negatively charged cell membrane. The DNA/polyelectrolyte complex formed interacts with the cell surface, and the DNA is then taken up by the cell, probably through lysosomes or endosomes.

Positively-charged, neutral and negatively-charged liposomes as well as positively-charged micelles have also been used for the delivery of nucleic acids to cells, in the micelle case relying on the positive charge of the micelles to provide a - "cross-budge" between the polyanionic nucleic acids and the polyanionic surfaces of the cells. For example, US-A-6,210,717 discloses the use of a mixed polymeric micelle containing an amphiphatic polyester-polycation copolymer and an amphiphatic polyester-sugar copolymer to deliver a selected nucleic acid into a host cell.

WO 02/082078 discloses the detection or quantification of analytes bound to immobilized capture molecules by affinity liposomes which contain encapsulated enzyme activators and surface-attached affinity components capable of specific binding to captured analytes. The enzyme activators are released from the liposomes by temperature- or detergent-mediated mechanisms to contact and activate solid phase-immobilized inactive enzyme molecules. In the presence of added substrate, the activated enzymes generate electrochemically or optically detectable reporter molecules which are measured.

WO 02/072873 discloses the preparation of a substrate surface supporting a lipid bilayer membrane by contacting a substrate surface with an aqueous liquid containing detergent/lipid mixed micelles to adhere detergent/lipid mixed micelles. The substrate surface is then contacted with an aqueous liquid substantially free from detergent to elute the detergent molecules from the adhered mixed micelles and make the remaining lipid molecules assemble into a lipid bilayer membrane on the substrate surface. A biomolecule, such as a membrane protein, may be included in the bilayer membrane by including the biomolecule in the mixed micelle preparation, or by attaching the biomolecule to the substrate surface prior to depositing the mixed micelle preparation.

It is an object of the present invention to overcome the problem of immobilizing oligonucleotides to a negatively charged surface.

### SUMMARY OF THE INVENTION

The above and other objects and advantages are provided by a novel method for immobilizing a target molecule to solid support surface wherein the target molecule is first complexed with a vesicular structure in the form of a liposome or micelle whereupon the complex is contacted with the solid support to permit the target molecule to bind to the surface.

The present invention therefore provides a method of immobilizing a target molecule to a solid support surface capable of binding the target molecule, wherein the target molecule and the solid support surface carry electric charges of the same kind wich method comprises the steps of:
complexing the target molecule with a Liposome or micelle capable of forming a dissociable complex with the target molecule and carrying an electric charge of a kind opposite to that of the target molecule,
contacting the complex formed with the solid support surface to thereby bind the target molecule to the surface and dissociate the complex, and
removing the Liposome or micelle from the solid support surface to leave the target molecule immobilized on the surface.

If the complex is not dissociated when binding to the solid support surface, a subsequent treatment or wash may be required.

The above and other aspects of the invention will be evident upon reference to the accompanying drawing and the following detailed description.

### BRIEF DESCRIPTION OF THE DRAWING

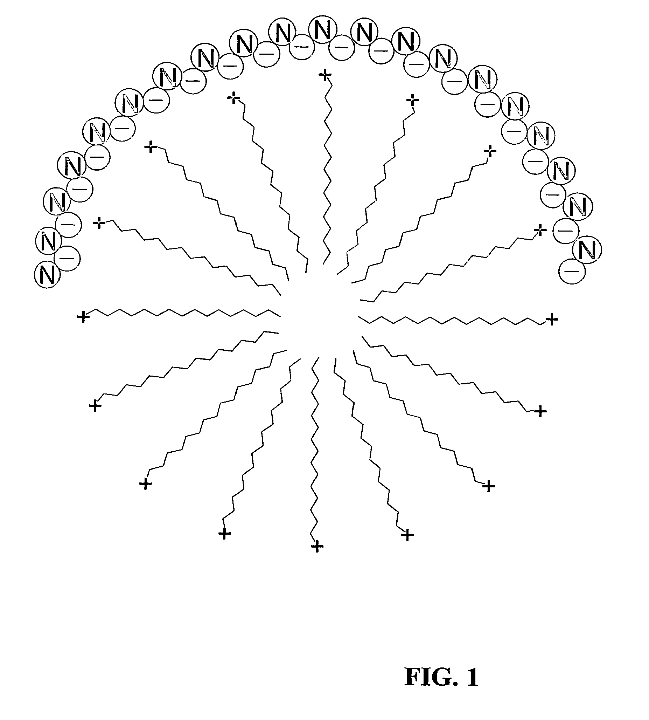

Figure 1 is a schematic illustration of a micalle/oligonucleotide complex.

### DEFINITIONS

In describing and claiming the present invention, the following terminology will be used in accordance with the definitions set out below.

"Solid support" refers to any solid (flexible or rigid) substrate onto which it is desired to immobilize one or more target compounds. The substrate may be biological, non-biological, organic, inorganic or a combination thereof, and may be in the form of particles, strands, precipitates, gels, sheets, tubings, spheres, containers, capillaries, pads, slices, films, plates, slides, etc, having any convenient shape, including disc, sphere, circle, etc.

"Complex" refers to a chemical association of two (or more) species joined usually by weak electrostatic forces. A complex is dissociable if it can be dissociated into the two complex-forming species.

"Vesicular structure" refers to an organized structure of amphiphatic (surfactant) molecules having both hydrophobic and hydrophilic domains, and includes, for example, liposomes, micelles or inverse micelles. Liposomes are spherical vesicles formed by a bilayer of lipids, usually phospholipids, that enclose an aqueous volume, whereas micelles and inverse micelles are colloidal aggregates of amphiphatic molecules, which occur at (and above) a well-defined concentration known as the critical micelle concentration (CMC). The typical number of aggregated molecules in a micelle is about 50 to 100.

"Functional group" refers to a reactive chemical entity. When present on a solid support surface, the functional group serves to connect a binding agent, such as a capture agent or a ligand, to the surface. Usually, functional groups need to be activated in order to immobilize a binding agent. The functional groups may be inherently present in the material used for the solid support or they may be provided by treating or coating the support with a suitable material containing the functional group. A functional group may also be introduced by reacting the solid support surface with an appropriate chemical agent.

"Activation" refers to a modification of a functional group to a reactive group enabling or improving coupling of a binding agent thereto.

"Ligand" refers to a molecule that has a known or unknown affinity for a given analyte. The ligand may be a naturally occurring molecule or one that has been synthesized. The ligand may be used *per se* or as aggregates with another species. Optionally, the ligand may also be a cell.

"Analyte" refers to a molecule, which may be a macromolecule, such as a polypeptide or a polynucleotide, or even a small molecule, the presence, amount and/or identity of which are to be determined, or which is to be characterized in other respects, such as e.g. its binding properties. The analyte is recognized by a particular ligand forming an analyte/ligand pair. Optionally, the ligand may also be a cell. As used herein, the term analyte also includes analyte analogues.

"Capture agent" refers to a binding agent that can be immobilized to a solid support surface and which can bind to another species, such as a ligand or a second capture agent.

"Specific binding pair"(abbreviated "sbp") refers to a pair of molecules (each being a member of a specific binding pair) which are naturally derived or synthetically produced. One of the pair of molecules has a structure (such as an area or cavity) on its surface that specifically binds to (and is therefore defined as complementary with) a particular structure (such as a spatial and polar organisation) of the other molecule, so that the pair have the property of binding specifically to each other. Examples of types of specific binding pairs are protein-protein, antigen-antibody, antibody-hapten, biotin-avidin, ligand-receptor (e.g. hormone receptor, peptide-receptor, enzyme-receptor), carbohydrate-protein, carbohydrate-lipid, lectin-carbohydrate, nucleic acid-nucleic acid (including e.g. PNA-PNA; PNA = peptide nucleic acid), histidine residue(s)-metal chelate.

"Antibody" refers to an immunoglobulin which may be natural or partly or wholly synthetically produced and also includes active fragments, including Fab antigen-binding fragments, univalent fragments and bivalent fragments. The term also covers any protein having a binding domain which is homologous to an immunoglobulin binding domain. Such proteins can be derived from natural sources, or partly or wholly synthetically produced. Exemplary antibodies are the immunoglobulin isotypes and the Fab, Fab', F(ab')₂, scFv, Fv, dAb, and Fd fragments.

"Nucleic acid" refers to a deoxyribonucleotide polymer (DNA) or ribonucleotide polymer (RNA) in either single- or double-stranded form, and also encompasses synthetically produced analogs that can function in a similar manner as naturally occurring nucleic acids. While natural nucleic acids have a phosphate backbone, artificial nucleic acids may contain other types of backbones, nucleotides or bases. These include, for instance, peptide nucleic acids (PNAs) as described in e.g. US-A-5,948,902 and the references cited therein; pyranosyl nucleic acids (p-NAs) as described in e.g. WO 99/15540 (p-RNAs), WO 99/15539 (p-RNAs), and WO 00/11011 (p-DNAs); locked nucleic acids (LNAs), as described in e.g. US-A-6,316,198; and phosphothionates and other variants of the phosphate backbone of native nucleic acids.

"Oligonucleotide" refers to single stranded nucleotide multimers of from about 5 to about 100 nucleotides (including synthetically produced analogs as described for "nucleic acid" above).

"Polynucleotide" refers to single stranded nucleotide multimers of from about 100 nucleotides (including synthetically produced analogs as described for "nucleic acid" above).

"Low molecular weight organic compound" refers to an organic compound having a molecular weight in the range of from about 100 to about 1000, usually from about 250 to about 800. Low molecular weight compounds are sometimes also referred to as "small molecules".

"Array" generally refers to a linear or two-dimensional array of discrete regions, each having a finite area, formed on a continuous surface of a solid support and supporting one or more binding agents, such as e.g. capture agents or ligands. Ordered arrays of nucleic acids, proteins, small molecules, cells or other substances on a solid support enable parallel analysis of complex biochemical samples. In a "microarray", the density of discrete regions, or spots, is typically at least 100/cm², and the discrete regions typically have a diameter in the range of about 10-1000 µm, usually about 10-500 µm and are separated from other regions in the array by about the same distance.

"Biosensor" usually refers to a device that uses a component for molecular recognition (for example a layer with immobilised antibodies) in conjunction with a solid state physicochemical transducer.

In the specification and the appended claims, the singular forms "a", "an", and "the" are meant to include plural reference unless it is stated otherwise. Also, unless defined otherwise, technical and scientific terms used herein have the same meaning as commonly understood to a person skilled in the art related to the invention.

### DETAILED DESCRIPTION OF THE INVENTION

As mentioned above, the present invention generally relates to the immobilization of a target molecule to a solid support surface. Solid supports with immobilized target molecules are used in various fields, including analytical and separation techniques. Immobilization of a target molecule to a solid support often involves binding, such as covalent binding, of the molecule to the solid support surface. Usually, the solid support has a reactive chemical group that can react with the target molecule. Sometimes, the target molecule carries an electric charge, and if the solid support surface carries an opposite charge, the target molecule will be attracted to the solid support surface, facilitating the molecular interaction with the surface. On the other hand, if the solid support surface carries the same kind of charge as that of the target molecule, it is readily seen that immobilization may be reduced or even substantially prevented. An example of the latter case is the immobilization of oligonucleotides (which generally carry a negative charge) to negatively charged surfaces.

According to the present invention, it has now been found that a charged target molecule, particularly a macromolecule, such as e.g. an oligonucleotide, may be efficiently immobilized to a solid support surface, even if the surface carries the same kind of charge as that of the target molecule, if the target molecule is first complexed with a vesicular structure carrying a charge opposite to that of the target molecule, and the complex formed is then contacted with the solid support surface.

Without being limited to any particular theory, it is assumed that the improved immobilization efficiency is due to the target molecule/vesicular structure complex permitting the target molecule to come sufficiently near the surface for it to interact therewith. This is in turn assumed to be due, on one hand, to the e.g. positively charged vesicular structure at least partially neutralizing the opposite, e.g. negative, charge of the target molecule, and, on the other hand, e.g. in the case of a macromolecule such as an oligonucleotide, compacting of the macromolecule by the vesicular structure. Compacting may also result in faster diffusion of the molecule to the surface as compared to the diffusion of the free molecule. With regard to the assumed favourable compaction of a target molecule, the formation of the target molecule/vesicular structure complex may also improve immobilization thereof to a neutral solid support surface. Reducing repulsion forces between molecules to be immobilized may also lead to higher immobilization densities.

The present inventive concept is generally applicable to any target molecule which can form a dissociable complex with a charged or uncharged vesicular structure. The target molecule is, however, preferably a biomolecule (including synthetically produced biomolecules and analogues thereto), such as e.g. nucleic acids (including e.g. plasmids), proteins, polypeptides, lipids, and carbohydrates. More specific examples of biomolecules are oligonucleotides, polynucleotides, antibodies and enzymes. The target molecule may also be a low molecular weight organic compound,

The vesicular structure is a liposome or a micelle, especially a micelle. Liposomes or micelles may be mixed liposomes or mixed micelles, i.e. containing two (or more) liposome and micelle forming components, respectively.

In one embodiment of the present invention, a nucleic acid, particularly a nucleotide, is immobilized to a negatively charged solid support surface using a positively charged micelle or liposome. A schematic illustration of a complex between an oligonucleotide (20-mer) and a positively charged micelle (CTAB) is shown in Figure 1.

In another embodiment of the invention, a protein, particularly an antibody, carrying a negative charge is immobilized to a negatively charged solid support surface using a positively charged micelle or liposome.

In yet another embodiment of the invention, a low molecular weight organic compound, such as adenosine triphosphate (ATP) or nitrilo tri-acetic acid (NTA), carrying a negative charge is immobilized to a negatively charged solid support surface by means of a positively charged micelle or liposome.

In still another embodiment, a positively charged target molecule is immobilized to a positively charged solid support surface using a negatively charged micelle or liposome.

The relative amounts of target molecules and micelles or liposomes to be used depend on *inter alia* the particular target molecule and micelle or liposome, respectively, but suitable ratios may readily be established by a person skilled in the art. Generally, ratios of target molecule to micelle or liposome (number of target molecules to number of micelles) from about 1:3 to about 3:1, preferably from about from 1:2 to about 2:1, especially about 1:1 may be used.

The preparations of micelles and liposomes are well known to the skilled person and need not be described in any detail herein. For general descriptions of methods therefor it may be referred to, for example, Szoka, F., Jr., and Papahadjopoulos, D., Annu.Rev. Biophys. Bioeng. 9, 457 (1980); and Schwendener, R. A., Ansanger, M., and Weder, H. G., Biochem. Biophys. Res. Commun., 100, 1055 (1981).

Examples of amphiphatic molecules, or surfactants, from which positively charged micelles may be prepared include dodecyltrimethylammonium bromide (DTAB), cetyltrimethylammonium bromide (CTAB) (other name: hexadecyltrimethylammonium bromide), benzyldimethylhexadecylammonium chloride, dimethyldioctadecylammonium bromide, dodecylethyldimethylammonium bromide, ethylhexadecyldimethylammonium bromide, trimethyl(tetradecyl)ammonium bromide, and thonzonium bromide.

Negatively charged micelles may, for example, be prepared from sodium dodecylsulphate (SDS).

Examples of molecules from which positively or negatively charged liposomes may be prepared include hexadecyldimethylammoniumpropane-1-sulphonate.

Usually, it is preferred that only a part of the electric charge of the micelle or liposome is neutralized by the target molecule, such that there will remain a residual charge after the complex formation to permit the complex to interact electrostatically with an oppositely charged solid support surface.

The complex between target molecule and the vesicular structure is usually formed spontaneously. When the complex is then contacted with the solid support, the target molecule interacts with the reactive functional group or other binding moiety on the surface, so that the target molecule is immobilized thereto. This may cause simultaneous dissociation of the complex. Usually, however, a treatment or wash with a suitable solution will be required to dissociate the complex and remove the vesicular structure or residues thereof from the surface. The necessary solutions/conditions therefor are either well known to or may readily be established by the skilled person.

The solid support is preferably a rigid structure and may comprise a substrate having a surface layer of a different material. While the solid support may be a particle, it is usually a surface of a larger entity, such as an inner surface of a well or receptacle, or a plate or slide. Exemplary of the latter kind are solid supports used for protein or DNA/RNA chips, as well as sensing surfaces in sensor devices, such as biosensors.

The surface of the solid support may be composed of a variety of materials, for example, polymers, plastics, resins, polysaccharides, silica or silica-based materials, carbon, metals, inorganic glasses, membranes, etc. A suitable surface is a metal film, e.g. gold, silver, or aluminium, preferably gold

To permit immobilization of target molecules, the solid support surface comprises groups or molecules capable of interacting with the target molecule. Such groups may be functional groups, e.g. hydroxy, carboxy, amino, formyl, hydrazide, carbonyl, epoxy or vinyl, which may form a covalent bond with a functional group on the target molecule. Usually, the functional group(s) on the surface or on the target molecule is (are) activated to a more reactive group(s) prior to reaction with the target molecule. For example, an aminonucleotide may be coupled to a surface-bound carboxy group activated to a N-hydroxysuccinimide ester group. It is to be noted that a surface with an activated functional group that is e.g. negatively charged before the activation but neutral after the activation may develop a negative charge during the immobilization process due to competing hydrolysis of the activated group.

Alternatively, the solid support surface carries one member of a specific binding pair (sbp), and the other member of the specific binding pair is on the target molecule. A commonly used sbp for immobilizing oligonucleotides is biotin-avidin (or streptavidin).

Especially in biosensor contexts, the solid support surface may be part of a flow cell, permitting the methods of the invention to be performed *in situ* in the flow cell. Examples of flow cells used in the biosensor field are described in e.g. US-A-5,492,840, 5,513,264 and WO 99/36766

As mentioned above, the target molecule may, for instance, be an analyte-binding ligand, or a capture agent capable of binding a ligand. Alternatively, the capture agent may bind a binding agent which in turn can bind a ligand. A single capture agent may be used to bind a single ligand to the surface, or to bind different ligands to different discrete areas of the surface to provide a ligand array. Alternatively, a ligand array may be formed by selectively binding different ligands to specific capture agents provided on respective discrete surface areas. Binding of a ligand to a solid support surface, such as a sensor surface, is often referred to as "sensitizing" the surface.

Examples of ligands include, without any limitation thereto, agonists and antagonists for cell membranes, toxins and venoms, viral epitopes, antigenic determinants, hormones and hormone receptors, steroids, peptides, enzymes, substrates, cofactors, drugs, lectins, sugars, oligonucleotides, oligosaccharides, proteins, glycoproteins, cells, cellular membranes, organelles, cellular receptors, vitamins, viral epitopes, and immunoglobulins, e.g. monoclonal and polyclonal antibodies.

Exemplary capture agents include nucleic acids and antibodies, especially oligonucleotides. A surface with a capture agent in the form of an oligonucleotide may, for example, be used to bind a ligand conjugated to a complementary oligonucleotide tag. In this way, different ligands may readily be immobilized to different discrete areas of the surface. As mentioned above, it is also possible to immobilize different oligonucleotides to different discrete areas in order to capture e.g. differently oligonucleotide-tagged ligands thereto. This will permit different ligand conjugates to be addressed to different areas, but the capture surface may still be regenerated by general regeneration conditions.

Usually, the oligonucleotides have a length of at least six bases. It is further preferred that the oligonucleotide pairs are completely complementary over at least a portion of their respective sequences. Completely complementary sequences are, however, preferred.

Heterobifunctional agents that may be used to prepare ligand/oligonucleotide conjugates, such as e.g. antibody/oligonucleotide conjugates, are well known to and may readily be selected by the skilled person. For examples of such heterobifunctional agents, it may be referred to, for instance, the above-mentioned US-A-5,648,213

Examples of analytes that may be assayed for include, without any restriction thereto, agonists and antagonists for cell membrane receptors, toxins and venoms, viral epitopes, hormones (e.g. opiates, steroids, etc), hormone receptors, peptides, enzymes, enzyme substrates, cofactors, drugs, lectins, sugars, oligonucleotides, oligosaccharides, proteins, and monoclonal antibodies. Assaying an analyte is not restricted to qualitative or quantitative determination of the analyte, but also includes, for example, studying its interaction with a ligand for other characterization of the analyte, such as determining binding properties, e.g. affinity and kinetic constants.

Methods for detecting the presence of bound analyte(s) on the surface may be chosen from a wide variety of detection techniques, including both photometric and non-photometric methods of detection, for example, marker-based techniques, where the analyte(s) or an analyte specific reagent(s) is (are) labelled, e.g. with a radiolabel, a chromophore, fluorophore, chemiluminescent moiety or a transition metal; as well as label-free techniques.

For many applications, the assays are performed with a biosensor. Biosensors may be based on a variety of detection methods. Typically such methods include, but are not limited to, mass detection methods, such as piezoelectric, optical, thermo-optical and surface acoustic wave (SAW) device methods, and electrochemical methods, such as potentiometric, conductometric, amperometric and capacitance methods. With regard to optical detection methods, representative methods include those that detect mass surface concentration, such as reflection-optical methods, including both internal and external reflection methods, angle, wavelength or phase resolved, for example ellipsometry and evanescent wave spectroscopy (EWS), the latter including surface plasmon resonance (SPR) spectroscopy, Brewster angle refractometry, critical angle refractometry, frustrated total reflection (FTR), evanescent wave ellipsometry, scattered total internal reflection (STIR), optical wave guide sensors, evanescent wave-based imaging such as critical angle resolved imaging, Brewster angle resolved imaging, SPR angle resolved imaging, and the like. Further, photometric methods based on, for example, evanescent fluorescence (TIRF) and phosphorescence may also be employed, as well as waveguide interferometers. Also atomic force microscopy (AFR)-based detection methods may be mentioned.

In the Examples below, a biosensor instrument based on surface plasmon resonance (SPR) detection at a gold surface was used, providing "real-time" binding interaction analysis between a surface bound ligand and an analyte of interest by mass-sensing at the surface. A detailed discussion of the technical aspects of this type of biosensor, as well as of the phenomenon of SPR, may be found in the above-mentioned US-A-5,313,264. More detailed information on matrix coatings for biosensor sensing surfaces is given in, for example, US-A-5,242,828 and US-A-5,436,161. In addition, a detailed discussion of the technical aspects of the biosensor chips used with the biosensor instrument may be found in the above-mentioned US-A-5,492,840.

In the following Examples, various aspects of the present invention are disclosed more specifically for purposes of illustration and not limitation.

### EXAMPLES

A BIACORE^{®} 3000 instrument (Biacore AB, Uppsala, Sweden) was used. In this instrument, a micro-fluidic system passes samples and running buffer through four individually detected flow cells (one by one or in series), with very high precision and with small sample volumes needed. As sensor chip was used Sensor Chip CM5 (Biacore AB, Uppsala, Sweden) which has a gold surface with a covalently linked carboxymethyl-modified dextran polymer hydrogel. Running buffer was HBS-N (10 mM HEPES pH 7.4 and 150 mM NaCl) (Biacore AB, Uppsala, Sweden). Due to the carboxy groups, the hydrogel has an anionic character. The output from the instrument is a "sensorgram" which is a plot of detector response (measured in "resonance units", RU) as a function of time. An increase of 1000 RU corresponds to an increase of mass on the sensor surface of approximately 1 ng/mm².

The following two (complementary) oligonucleotides were used (chemically modified at the 5'-end with an amino group):
5' TTT CCT CAG CAT CTT ATC CG3', referred to as "BC1"
5' CGG ATA AGA TGC TGA GGA AA3', referred to as "BC2"

The nucleotide sequence BC1 is disclosed in Persson, B., et al. (1997) Anal. Biochem. 246, 34-44.

### Example 1 (Comparative)

### Immobilization of amino-modified oligonucleotide BC1 at high salt concentrations

Sensor Chip CM5 was activated by 0.2 M *N*-ethyl-*N*-dimethylamino-propylcarbodiimide (EDC) and 50 mM *N*-hydroxysuccinimide (NHS) for 7 or 20 minutes at a flow of 5 µl/min (EDC and NHS were from Biacore AB, Uppsala, Sweden), converting a fraction of the carboxyl groups on the dextran to reactive N-hydroxysuccinimide ester groups. 100 µM of amine-modified oligonucleotide BC1 (SGS DNA, Köping, Sweden, or DNA Technology, Ålborg, Denmark) in borate 8.5 immobilization buffer (Biacore AB, Uppsala, Sweden) was then injected for 13 minutes at 5 µl/min for immobilization thereof to the surface. Unreacted N-hydroxysuccinimide ester groups were deactivated by injecting ethanolamine for 3 minutes at 5 µl/min (replacing the activated group by hydroxyethylamide). The immobilization procedure was performed at high NaCl concentrations varying from 1 to 3 M to shield off the negative charges of the oligonucleotide, and at relatively high pH values between 7.0 and 8.5 to obtain a rapid coupling to the surface.

After each immobilization, the surface was washed with three pulses of 50 mM NaOH, 1 M NaCI for 1 min (flow 5 p,l/min) to remove loosely bound oligonucleotide. The complementary amino-modified oligonucleotide BC2 as well as a non-complementary oligonucleotide (BC1) were then allowed to hybridise to the immobilized amino-nucleotide BC1 for three minutes at a flow of 5 µl/min with HBS-EP buffer (Biacore AB, Uppsala, Sweden). Oligonucleotide concentrations varied between 1 and 10 µM. Regeneration of the surface between hybridizations was performed with 50 mM NaOH, 1 M NaCl for 1 minute.

The results are shown in Table 1 below.

**Table 1**

| pH | NaCl conc. (M) | NHS/EDC Activation time (min) | Immobilization (RU) | Hybridization of complementary oligo (RU) | Hybridization non-complementary oligo (RU) |
|---|---|---|---|---|---|
| 7.0 | 1 | 7 | -26 | 9.7 | 4.8 |
| 7.0 | 3 | 7 | -5.4 | 1.9 | 7.5 |
| 8.5 | 1 | 7 | 16.5 | 8.6 | 6.9 |
| 8.5 | 3 | 7 | 40.8 | 10.1 | 6.8 |
| 7.0 | 1 | 20 | 74.2 | 11.6 | 10.1 |
| 7.0 | 3 | 20 | -28.9 | 3.2 | 8.4 |
| 8.5 | 1 | 20 | -7.8 | 3.7 | 7.3 |
| 8.5 | 3 | 20 | 15.0 | 4.4 | - |

As appears from the Table, a high salt concentration was not effective to increase immobilization of the amino-oligonucleotide to the surface. Neither was the immobilization influenced by varying the pH or increasing the NHS/EDC activation time.

### Example 2 (comparative)

### Immobilization of amino-modified oligonucleotide BC1 with presence of tetramethylammonium chloride

Following the same immobilization protocol as in Example 1, oligonucleotide BC1 was immobilized to a CM5 chip surface, except that HBS-EP (Biacore AB, Uppsala, Sweden) was used as immobilization buffer, and 1M, 0.75 M, 0.5 M or 0.25 M tetramethylammonium chloride (TMA-Cl) was used instead of NaCl to shield off the negative charges of the oligonucleotide. After washing with 50 mM NaOH, 1 M NaCl, hybridizations with complementary and non-complementary oligonucleotides were then performed as described in Example 1. The results are shown in Table 2 below.

**Table 2**

| TMA-Cl conc (M) | Immobilization (RU) | Hybridization of complementary oligo (RU) | Hybridization of non-complementary oligo (RU) |
|---|---|---|---|
| 0.25 | -0.9 | 6.7 | 4.5 |
| 0.50 | -4.1 | 3.8 | 4.9 |
| 0.75 | -1.7 | 3.5 | 4.8 |
| 1.00 | 3.5 | 3.3 | 4.3 |

As appears from the Table, TMA-Cl had no effect on the immobilization of oligonucleotide to the surface.

### Example 3

### Immobilization of amino-modified oligonucleotide BC1 through complexing with cetyltrimethylammonium bromide micelles

Sensor Chip CM5 was activated as described in Example 1 above. 10-50 µM of amine-modified oligonucleotide BC1 (SGS DNA, Köping, Sweden, or DNA Technology, Ålborg, Denmark) in 10 mM Hepes pH 7.4 (Biacore AB, Uppsala, Sweden) with varying concentrations of cetyltrimethylammonium bromide (CTAB) were then injected for 10 minutes at 5 µl/min for immobilization thereof to the surface. Unreacted N-hydroxysuccinimide ester groups were deactivated by injecting ethanolamine for 3 minutes at 5 µl/min. After washing with 50 mM NaOH, 1 M NaCl, hybridizations with complementary and non-complementary oligonucleotides were then performed as described in Example 1. The results are shown in Table 3 below.

**Table 3**

| CTAB conc. (mM) | Amino-oligo conc. (µM) | Immobilization (RU) | Loss at wash (RU) | Hybridization of complementary oligo | Hybridization of non-complementary oligo |
|---|---|---|---|---|---|
| 0.40 | 10 | 3170 | -690 | 2181 | -0.6 |
| 0.60 | 10 | 3440 | -649 | 2701 | 2.0 |
| 0.80 | 10 | 3322 | -612 | 2390 | 1.8 |
| 1.00 | 10 | 2763 | -527 | 1877 | 0.8 |
| 0.75 | 25 | 3075 | -423 | 1569 | -1.3 |
| 1.50 | 25 | 3175 | -474 | 2375 | 2.1 |
| 2.25 | 25 | 3170 | -500 | 2317 | 2.3 |
| 3.00 | 25 | 3046 | -511 | 2258 | 2.2 |
| 2.25 | 50 | 3121 | -511 | 2388 | -1.5 |
| 3.00 | 50 | 3147 | -471 | 2349 | 1.4 |
| 3.75 | 50 | 3103 | -442 | 2312 | 2.5 |
| 4.50 | 50 | 2863 | -446 | 2242 | 2.8 |

As can be seen in the Table, high immobilization and hybridisation levels were obtained. The most suitable concentration of CTAB apparently varies with the concentration of the oligonucleotide BC1. The critical micellar concentration (CMC) for CTAB in the immoblization buffer used is likely to be near 0.29 mM. Since an average micelle of CTAB contains 61 molecules, the Table indicates an optimum micelle:oligo ratio of approximately 1:1 (0.60 mM CTAB/10 µM BC1; 1.50 mM CTAB/25 µM BC1; and 3.00 mM CTAB/50 µM BC1, respectively).

### Example 4

### Immobilization of amino-modified oligonucleotide BC1 through complexing with dodecyltrimethylammonium bromide micelles

The procedure described in Example 3 was followed, except that dodecyltrimethylammonium bromide (DTAB) was substituted for cetyltrimethylammonium bromide (CTAB). No hybridization with non-complementary oligonucleotide was performed. The results obtained are shown in Table 4 below.

**Table 4**

| DTAB conc. (mM) | Amino-oligo conc. (µM) | Immobilization (RU) | Loss at wash (RU) | Hybridization of complementary oligo |
|---|---|---|---|---|
| 0 | 10 | 10.5 | -116.6 | 15 |
| 5 | 10 | 3054.5 | -625.8 | 1970.7 |
| 10 | 10 | 3372.7 | -680.4 | 2054.1 |
| 15 | 10 | 3519.7 | -625.4 | 2276.5 |

## Claims

1. A method of immobilizing a target molecule to a solid support surface capable of binding the target molecule, wherein the target molecule and the solid support surface carry electric charges of the same kind, which method comprises the steps of:
complexing the target molecule with a liposome or a micelle capable of forming a dissociable complex with the target molecule and carrying an electric charge of a kind opposite to that of the target molecule,
contacting the complex formed with the solid support surface to thereby bind the target molecule to the surface,
dissociating the complex, and
removing the liposome or micelle from the solid support surface to leave the target molecule immobilized on the surface.

2. The method according to claim 1, wherein the target molecule is complexed with a micelle.

3. The method according to claim 1 or 2, wherein the target molecule and the solid support surface each carry a negative charge.

4. The method according to any one of claims 1 to 3, wherein the target molecule is a ligand capable of binding an analyte.

5. The method according to any one of claims 1 to 3, wherein the target molecule is a capture agent capable of binding a ligand or a ligand-binding agent.

6. The method according to any one of claims 1 to 5, wherein the target molecule is a nucleic acid or an antibody, preferably an oligonucleotide.

7. The method according to claim 6, wherein the target molecule is an artificial nucleic acid, preferably an artificial oligonucleotide.

8. The method according to any one of claims 1 to 5, wherein the target molecule is a low molecular weight organic compound.

9. The method according to any one of claims 1 to 8, wherein the solid support surface comprises a reactive group capable of reacting with a functional group of the target molecule to form a covalent bond.

10. The method according to any one of claims 1 to 8, wherein the solid support surface comprises one member of a specific binding pair, and the other member of the binding pair is conjugated to or part of the target molecule.

11. The method of claim 10, wherein the surface-bound member is avidin or streptavidin, and the target molecule is biotin-tagged.

12. The method according to any one of claims 1 to 11, wherein the solid support surface comprises a hydrogel, preferably a hydrogel based on dextran.

13. The method according to claim 12, wherein the dextran comprises carboxymethyl groups.

14. The method according to claim 13, wherein the carboxymethyl groups are activated to reactive groups.

15. The method according to any one of claims 1 to 14, wherein the ratio of target molecule to micelle or liposome is about 1:1.

16. The method according to any one of claims 1 to 15, wherein the micelle comprises cetyltrimethylammonium bromide (CTAB).

17. The method according to any one of claims 1 to 16, wherein the method is carried out in a flow cell.

18. The method according to any one of claims I to 17, wherein the solid support is a sensor surface.

19. The method according to claim 18, wherein the sensor surface permits detection of events at the surface by mass-sensing, preferably evanescent wave-based, e.g. SPR.

20. The method according to any one of claims 1 to 19, wherein the solid support is a chromatographic particle.

21. The method according to any one of claims 5 to 20, wherein the target molecule is a capture agent capable of binding a ligand, and the method comprises the additional step of contacting the surface with the ligand to bind the ligand to the immobilized capture agent.

22. The method according to claim 21, wherein the capture agent is an oligonucleotide, and the ligand is conjugated to an oligonucleotide complementary to the capture oligonucleotide.

23. The method according to claim 21 or 22, wherein different discrete areas of the solid support surface supporting a general capture agent are selectively contacted with different ligands to provide a solid support surface with an array of different ligands.

24. The method according to claim 21 or 22, wherein different discrete areas of the solid support surface, each supporting a different capture agent, are contacted with different ligands to provide a solid support surface with an array of different ligands.

## Patentansprüche

1. Verfahren zum Immobilisieren eines Zielmoleküls an einer Oberfläche eines festen Trägers, die zum Binden des Zielmoleküls fähig ist, wobei das Zielmolekül und die Oberfläche des festen Trägers elektrische Ladungen derselben Art tragen, wobei das Verfahren die Schritte umfasst:
Komplexieren des Zielmoleküls mit einem Liposom oder einer Mizelle, die fähig sind, einen dissoziierbaren Komplex mit dem Zielmolekül zu bilden und
eine elektrische Ladung einer Art zu tragen, die zu jener des Zielmoleküls entgegengesetzt ist,
In-Kontakt-Bringen des gebildeten Komplexes mit der Oberfläche des festen Trägers, um **dadurch** das Zielmolekül an die Oberfläche zu binden,
Dissoziieren des Komplexes, und
Entfernen des Liposoms oder der Mizelle von der Oberfläche des festen Trägers, um das Zielmolekül auf der Oberfläche immobilisiert zu hinterlassen.

2. Verfahren nach Anspruch 1, wobei das Zielmolekül mit einer Mizelle komplexiert wird.

3. Verfahren nach Anspruch 1 oder 2, wobei das Zielmolekül und die Oberfläche des festen Trägers jeweils eine negative Ladung tragen.

4. Verfahren nach einem der Ansprüche 1 bis 3, wobei das Zielmolekül ein Ligand ist, der fähig ist, einen Analyten zu binden.

5. Verfahren nach einem der Ansprüche 1 bis 3, wobei das Zielmolekül ein Einfangmittel ist, das fähig ist, einen Liganden oder ein Liganden-bindendes Mittel zu binden.

6. Verfahren nach einem der Ansprüche 1 bis 5, wobei das Zielmolekül eine Nukleinsäure oder ein Antikörper ist, bevorzugt ein Oligonukleotid.

7. Verfahren nach Anspruch 6, wobei das Zielmolekül eine künstliche Nukleinsäure ist, bevorzugt ein künstliches Oligonukleotid.

8. Verfahren nach einem der Ansprüche 1 bis 5, wobei das Zielmolekül eine organische Verbindung eines niedrigen Molekulargewichts ist.

9. Verfahren nach einem der Ansprüche 1 bis 8, wobei die Oberfläche des festen Trägers eine reaktive Gruppe umfasst, die fähig ist, mit einer funktionellen Gruppe des Zielmoleküls zu reagieren, um eine kovalente Bindung zu bilden.

10. Verfahren nach einem der Ansprüche 1 bis 8, wobei die Oberfläche des festen Trägers ein Element eines spezifischen Bindungspaars umfasst, und das andere Element des Bindungspaars an das Zielmolekül konjugiert ist oder ein Teil davon ist.

11. Verfahren nach Anspruch 10, wobei das an die Oberfläche gebundene Element Avidin oder Streptavidin ist, und das Zielmolekül mit einem Biotin-Tag versehen ist.

12. Verfahren nach einem der Ansprüche 1 bis 11, wobei die Oberfläche des festen Trägers ein Hydrogel umfasst, bevorzugt ein Hydrogel basierend auf Dextran.

13. Verfahren nach Anspruch 12, wobei das Dextran Carboxymethyl-Gruppen umfasst.

14. Verfahren nach Anspruch 13, wobei die Carboxymethyl-Gruppen aktiviert werden zu reaktiven Gruppen.

15. Verfahren nach einem der Ansprüche 1 bis 14, wobei das Verhältnis des Zielmoleküls zur Mizelle oder zum Liposom ungefähr 1:1 beträgt.

16. Verfahren nach einem der Ansprüche 1 bis 15, wobei die Mizelle Cetyltrimethylammoniumbromid (CTAB) umfasst.

17. Verfahren nach einem der Ansprüche 1 bis 16, wobei das Verfahren in einer Fließzelle ausgeführt wird.

18. Verfahren nach einem der Ansprüche 1 bis 17, wobei der feste Träger eine Sensoroberfläche ist.

19. Verfahren nach Anspruch 18, wobei die Sensoroberfläche eine Detektion von Ereignissen an der Oberfläche ermöglicht durch Massendetektion, bevorzugt basierend auf einer evaneszenten Welle, z. B. SPR.

20. Verfahren nach einem der Ansprüche 1 bis 19, wobei der feste Träger ein chromatographisches Teilchen ist.

21. Verfahren nach einem der Ansprüche 5 bis 20, wobei das Zielmolekül ein Einfangmittel ist, das zum Binden eines Liganden fähig ist, und das Verfahren umfasst den zusätzlichen Schritt des In-Kontakt-Bringens der Oberfläche mit dem Liganden, um den Liganden an das immobilisierte Einfangmittel zu binden.

22. Verfahren nach Anspruch 21, wobei das Einfangmittel ein Oligonukleotid ist, und der Ligand konjugiert wird an ein Oligonukleotid, das zum Einfang-Oligonukleotid komplementär ist.

23. Verfahren nach Anspruch 21 oder 22, wobei verschiedene getrennte Gebiete der Oberfläche des festen Trägers, die ein allgemeines Einfangmittel trägt, selektiv in Kontakt gebracht werden mit verschiedenen Liganden, um eine Oberfläche eines festen Trägers mit einem Feld verschiedener Liganden bereitzustellen.

24. Verfahren nach Anspruch 21 oder 22, wobei verschiedene getrennte Gebiete der Oberfläche des festen Trägers, die jeweils ein unterschiedliches Einfangmittel tragen, in Kontakt gebracht werden mit verschiedenen Liganden, um eine Oberfläche eines festen Trägers bereitzustellen mit einem Feld verschiedener Liganden.

## Revendications

1. Procédé permettant d'immobiliser une molécule cible sur une surface de support solide capable de se lier à la molécule cible, dans lequel la molécule cible et la surface de support solide portent des charges électriques identiques, le procédé comprenant les étapes consistant à :
mettre en complexe la molécule cible avec un liposome ou une micelle capable de constituer un complexe dissociable avec la molécule cible et portant une charge électrique opposée à celle de la molécule cible,
mettre en contact le complexe ainsi obtenu avec la surface de support solide afin de lier la molécule cible à la surface,
dissocier le complexe, et
supprimer le liposome ou la micelle de la surface de support solide pour laisser la molécule cible immobilisée sur la surface.

2. Procédé selon la revendication 1, dans lequel la molécule cible est mise en complexe avec une micelle.

3. Procédé selon la revendication 1 ou 2, dans lequel la molécule cible et la surface de support solide portent chacune une charge négative.

4. Procédé selon l'une quelconque des revendications 1 à 3, dans lequel la molécule cible est un ligand capable de se lier à un analyte.

5. Procédé selon l'une quelconque des revendications 1 à 3, dans lequel la molécule cible est un agent de capture capable de se lier à un ligand ou un agent capable de se lier à un ligand.

6. Procédé selon l'une quelconque des revendications 1 à 5, dans lequel la molécule cible est un acide nucléique ou un anticorps, de préférence un oligonucléotide.

7. Procédé selon la revendication 6, dans lequel la molécule cible est un acide nucléique artificiel, de préférence un oligonucléotide artificiel.

8. Procédé selon l'une quelconque des revendications 1 à 5, dans lequel la molécule cible est un composé organique de faible poids moléculaire.

9. Procédé selon l'une quelconque des revendications 1 à 8, dans lequel la surface de support solide comprend un groupe réactif capable de réagir avec un groupe fonctionnel de la molécule cible pour former une liaison covalente.

10. Procédé selon l'une quelconque des revendications 1 à 8, dans lequel la surface de support solide comprend un élément d'une paire spécifique de liaison, et l'autre élément de la paire de liaison est conjugué à la molécule cible ou à une partie de celle-ci.

11. Procédé selon la revendication 10, dans lequel l'élément lié à la surface est l'avidine ou la streptavidine, et la molécule cible est marquée par la biotine.

12. Procédé selon l'une quelconque des revendications 1 à 11, dans lequel la surface de support solide comprend un hydrogel, de préférence un hydrogel à base de dextrane.

13. Procédé selon la revendication 12, dans lequel le dextrane comprend des groupes carboxyméthyle.

14. Procédé selon la revendication 13, dans lequel les groupes carboxyméthyle sont activés en des groupes réactifs.

15. Procédé selon l'une quelconque des revendications 1 à 14, dans lequel le rapport de la molécule cible à la micelle ou au liposome est d'environ 1 :1.

16. Procédé selon l'une quelconque des revendications 1 à 15, dans lequel la micelle comprend le bromure de cétyltriméthylammonium (CTAB).

17. Procédé selon l'une quelconque des revendications 1 à 16, dans lequel le procédé est conduit dans une cuve de circulation.

18. Procédé selon l'une quelconque des revendications 1 à 17, dans lequel le support solide est une surface de capteur.

19. Procédé selon la revendication 18, dans lequel la surface de capteur permet de détecter des événements au niveau de la surface par détection de masse, de préférence fondée sur les ondes évanescentes, comme par exemple la résonance plasmonique de surface (SPR).

20. Procédé selon l'une quelconque des revendications 1 à 19, dans lequel le support solide est une particule chromatographique.

21. Procédé selon l'une quelconque des revendications 5 à 20, dans lequel la molécule cible est un agent de capture capable de se lier à un ligand, et le procédé comprend l'étape supplémentaire consistant à mettre en contact la surface avec le ligand pour lier le ligand à l'agent de capture immobilisé.

22. Procédé selon la revendication 21, dans lequel l'agent de capture est un oligonucléotide, et le ligand est conjugué à un oligonucléotide complémentaire de l'oligonucléotide de capture.

23. Procédé selon la revendication 21 ou 22, dans lequel différentes zones discrètes de la surface de support solide portant un agent de capture général sont sélectivement mises en contact avec différents ligands pour produire une surface de support solide comportant un réseau de différents ligands.

24. Procédé selon la revendication 21 ou 22, dans lequel différentes zones discrètes de la surface de support solide, portant chacune un agent de capture différent, sont mises en contact avec différents ligands pour produire une surface de support solide comportant un réseau de différents ligands.
